Europäisches Patentamt

European Patent Office

Office européen des brevets

(19)

(11) Numéro de publication : **0 054 045**
**B1**

# (12) FASCICULE DE BREVET EUROPÉEN

(45) Date de publication du fascicule du brevet :
**20.08.86**

(21) Numéro de dépôt : **81901690.8**

(22) Date de dépôt : **23.06.81**

(86) Numéro de dépôt international :
**PCT/FR 81/00082**

(87) Numéro de publication internationale :
**WO/8200061 (07.01.82 Gazette 82/01)**

(51) Int. Cl.⁴ : **G 01 S   7/52, G 01 S 15/89,
G 10 K 11/34, A 61 B 10/00**

(54) **DISPOSITIF RECEPTEUR POUR ECHOGRAPHE A SONDE ULTRASONORE MULTI-ELEMENTS.**

(30) Priorité : **23.06.80 DE 3023386**

(43) Date de publication de la demande :
**23.06.82 Bulletin 82/25**

(45) Mention de la délivrance du brevet :
**20.08.86 Bulletin 86/34**

(84) Etats contractants désignés :
**FR**

(56) Documents cités :
**US-A- 4 019 169**
**US-A- 4 159 462**
**US-A- 4 173 007**
**Le dossier contient des informations techniques présentées postérieurement au dépôt de la demande et ne figurant pas dans le présent fascicule.**

(73) Titulaire : **THOMSON-CSF**
**173, Boulevard Haussmann**
**F-75379 Paris Cedex 08 (FR)**

(72) Inventeur : **DEMUTH, Dieter**
**Zum Mülenberg 14**
**D-5840 Schwerte 5 (DE)**

(74) Mandataire : **Thrierr, Françoise et al**
**THOMSON-CSF SCPI 19, avenue de Messine**
**F-75008 Paris (FR)**

Jouve, 18, rue St-Denis, 75001 Paris, France

## Description

La présente invention concerne un dispositif récepteur pour échographe à sonde ultrasonore multi-éléments. Elle concerne plus particulièrement le domaine du diagnostic médical ultrasonore. Il est connu dans l'art antérieur des dispositifs piézoélectriques émetteur-récepteurs. Ces dispositifs ou sondes piézoélectriques permettent d'émettre des ondes ultrasonores à l'excitation des cristaux piézoélectriques par des impulsions électriques à une fréquence d'émission prédéterminée. Les ondes émises se propagent dans le milieu dont on cherche à réaliser une image. Elles sont réfléchies en partie par des différences d'impédance acoustiques entre les milieux inhomogènes rencontrés. Les réflexions sont appelées échos. Ce sont des ondes de pression que la sonde piézoélectrique traduit en impulsion électrique dit signaux d'échos de réponse. L'onde ultrasonore ayant une certaine célérité de propagation dans le milieu, chaque écho est reçu après l'émission à une date qui dépend de la distance à l'obstacle qui le renvoie. Pour réaliser des explorations automatiques de l'intérieur d'un corps par le moyen d'ultrasons, il a été proposé d'émettre des ondes ultrasonores par des sondes multi-éléments. Dans de telles sondes, plusieurs éléments transducteurs piézoélectriques sont répartis. Le faisceau ultrasonore est formé à l'émission en excitant chaque élément transducteur en relation de phase avec ses voisins.

Dans une première réalisation dite sonde à balayage linéaire, la sonde comporte N éléments adjacents. A un instant donné, le faisceau ultrasonore est engendré par l'excitation de P éléments adjacents pris parmi les N éléments de la sonde. En appliquant des retards de temps prédéterminés, variables d'un élément à l'autre, il est possible de focaliser l'énergie ultrasonore du faisceau en un point déterminé dit foyer d'examen. En déplaçant l'adressage des signaux électriques à la série de P éléments adjacents, il est possible de réaliser un balayage linéaire du plan d'examen.

Dans une deuxième application dite de sonde à balayage sectoriel, la loi de retard de temps appliquée à chaque élément transducteur de la sonde est telle que le foyer obtenu peut être déplacé angulairement autour de l'axe de la sonde.

A la réception, il est prévu que chaque signal d'écho reçu soit transmis au dispositif de traitement du signal à travers une ligne à retard réglable par le moyen de circuit électronique de commande. Pour obtenir de grandes dynamiques de fonctionnement, il est nécessaire que chaque ligne à retard soit capable de fournir des retards aussi petits que possible et des retards dépassant la seconde. A chaque instant les lois de retard appliquées à ces éléments de ligne à retard doivent être telles que les diférents signaux transmis au dispositif de traitement correspondent à un moment donné à un même écho réfléchi par l'obstacle.

Pour réaliser ces lignes à retard, il est connu d'utiliser des éléments à couplage de charge dits CCD (Charge Coupled Device).

Dans un tel dispositif, tout signal présenté à l'entrée est transmis progressivement à un certain rythme d'analyse d'entrée et à une cadence de traversée qui sont déterminés par un signal d'horloge pouvant varier dans de larges domaines de fréquence qui déterminent la dynamique et donc les caractéristiques du faisceau ultrasonore reçu.

Pour obtenir des retards différents pour chaque canal de ligne à retard, la fréquence d'horloge est diversifiée en fonction de l'angle de réception et donc de l'élément transducteur concerné. Le réglage des fréquences d'horloge doit se faire rapidement et avec une précision donnée. Les circuits électroniques nécessaires pour une telle réalisation entraînent une élévation du coût du matériel d'examen. C'est le cas en particulier où le réglage de la fréquence se fait dans un synthétiseur au moyen d'un oscillateur commandé par tension VCO. Un autre problème posé par les dispositifs de service des lignes à retard est à la dérive en température. Enfin, il faut aussi compenser la dépendance en tension.

D'autre part, le brevet américain 4 173 007 décrit un système à CCD tel que défini dans le préambule de la revendication 1 dans lequel il est suggéré de charger et décharger ces composants au moyen d'une horloge connectable et déconnectable sélectivement au cours des cycles émission-réception successifs. Cependant, les moyens de couplage entre l'horloge et les composants CCD n'y sont pas décrits.

L'invention se rapporte à un dispositif récepteur du genre mentionné en dernier lieu dans l'introduction.

L'invention concerne donc un dispositif récepteur pour échographe comprenant une sonde ultrasonore comportant une pluralité d'éléments transducteurs, un nombre correspondant d'unités d'éléments à transfert de charge interconnectés respectivement entre chaque transducteur et un moyen sommateur, lesdites unités étant destinées à emmagasiner des signaux d'écho délivrés selon une séquence de réception prédéterminée par les éléments transducteurs, et un circuit de contrôle pilotant la mémorisation et la lecture des signaux d'écho emmagasinés et déterminant, pour chaque unité, un délai de déclenchement de la mémorisation, ledit circuit comportant une horloge, caractérisé en ce que pour chaque unité le circuit de contrôle comporte un moyen retardateur, une porte et un compteur de longueur de ligne déterminant la durée pendant laquelle les signaux d'écho sont admis à l'unité associée, chaque moyen retardateur étant connecté entre ladite horloge et la porte associée dont la sortie est reliée à une entrée de commande de l'unité associée ainsi qu'au compteur de longueur de ligne associé, le circuit de contrôle comportant en outre un circuit de commande opératoire délivrant des codes numériques pour programmer à chaque séquence, d'une part, chacun desdits moyens retardateurs et, d'autre part, chacun desdits compteurs de longueur de ligne, chaque moyen retardateur et chaque compteur de longueur de ligne comportant à cet effet des entrées de programmation respectives, lesdits codes numériques appliqués à chaque séquence étant respectivement représentatifs de l'instant initial de chargement des éléments à transfert de charge de ladite unité associée et de la durée des signaux d'écho à emmagasiner dans lesdits éléments, et une porte de validation aux entrées de laquelle les sorties de tous les compteurs de longueur de ligne sont reliées, ainsi que des moyens de lecture simultanée des éléments à transfert de charge préalablement utilisés en chargement-écriture, lesdits moyens de lecture étant pilotés par la porte de validation.

Les particularités et avantages de l'invention vont ressortir de la description qui va suivre du principe schématisé et mis en œuvre et d'un mode avantageux de réalisation donné à titre d'exemple non limitatif en référence aux dessins qui représentent :

figure 1 un schéma de montage d'un circuit selon l'invention,

figure 2 une variante de réalisation du circuit de la figure 1.

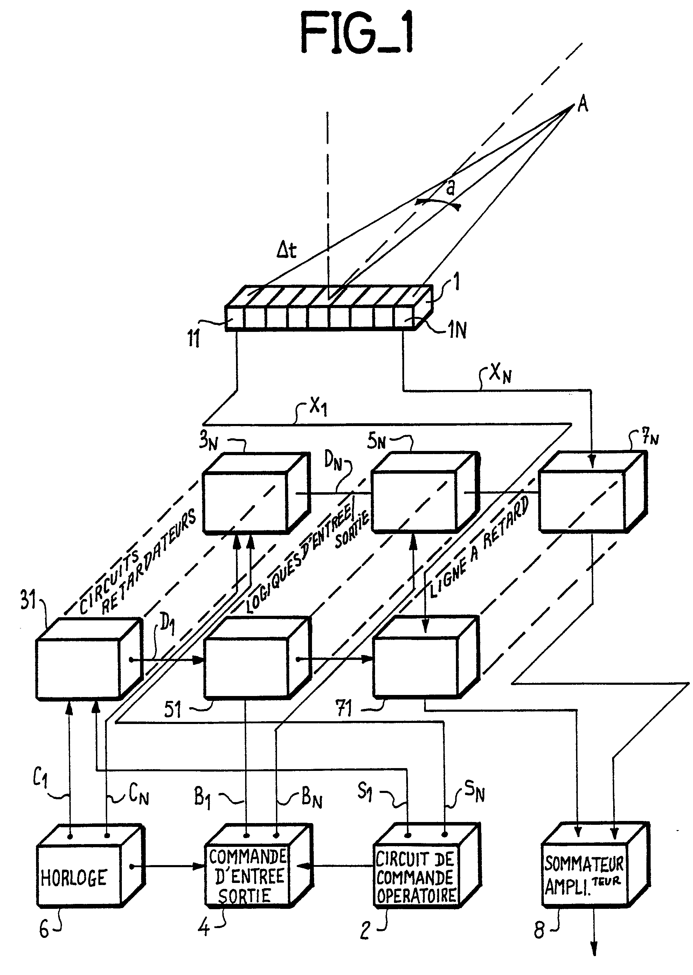

A la figure 1, une sonde 1 comporte une pluralité d'éléments transducteurs 11 qui, excités par un générateur d'impulsions comportant autant de voies que de transducteurs, émettent une onde ultrasonore focalisée au point A. Cette sonde constitue un faisceau incliné sous l'angle a avec la perpendiculaire à la sonde linéaire. Selon la loi des retards du signal appliqué à chaque élément transducteur, l'angle a et la distance du point A au centre de la sonde varie. Le générateur d'impulsions d'excitation de la sonde, connu en lui-même, n'a pas été représenté au dessin. L'onde concentrée au point A est réémis en partie par celui-ci et revient sous forme d'écho ultrasonore sur les éléments transducteurs 11 à 1N. L'élément transducteur, en réponse à l'onde ultrasonore qu'il reçoit, engendre un signal dit signal d'écho X1 à XN qui est transmis à un sommateur-amplificateur 8 à travers une ligne à retard 71 à 7N. Les retards sur la ligne à retard sont commandés par un circuit retardateur 31 à 3N. Chaque circuit retardateur 31 à 3N reçoit d'une part une commande de cadencement C1 à CN d'une horloge 6 et d'autre part, une commande opératoire S1 à SN d'un circuit de commande opératoire 2. Chaque ligne à retard 71 à 7N reçoit la commande D1 à DN des signaux retardateurs 31 à 3N par l'intermédiaire des logiques d'entrée-sortie 51 à 5N. Chacune de ces logiques 51 à 5N reçoit une commande B1 à BN d'un circuit de commande d'entrée-sortie 4.

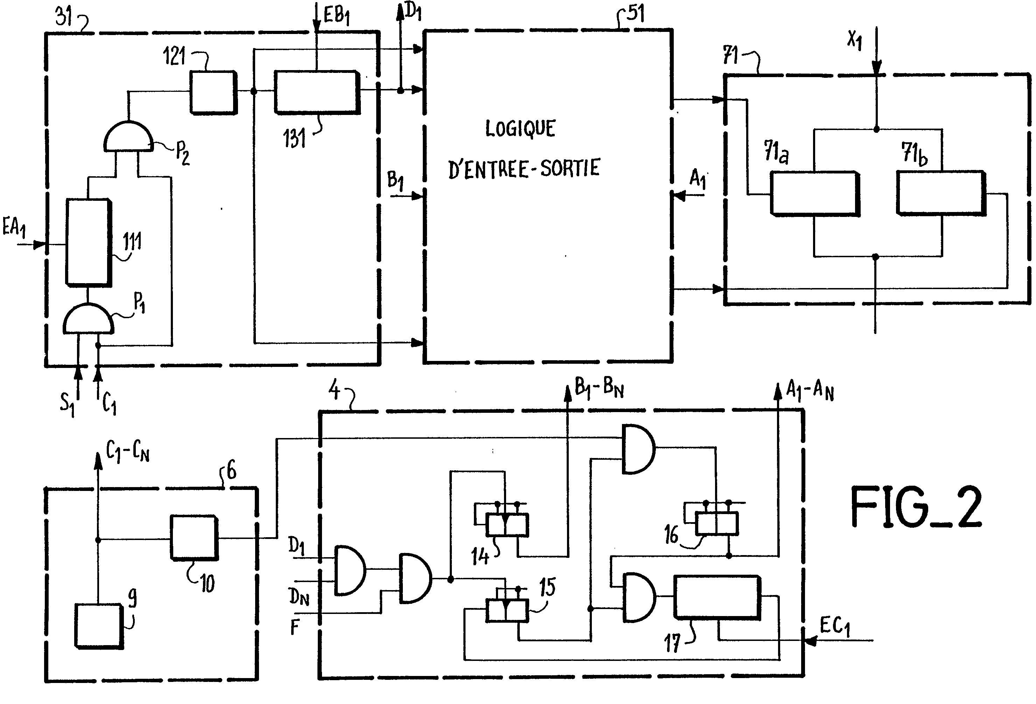

A la figure 2 a été représenté un mode de réalisation particulier selon l'invention. Sur cette figure a été représenté un canal d'acquisition de données recevant un signal d'écho X1 transmis au sommateur-amplificateur 8. Un canal comporte une ligne à retard 71, une logique d'entrée-sortie 51, un circuit retardateur 31 qui reçoit les commandes du circuit de commande opératoire 2 non représenté ici, un circuit de commande d'entrée-sortie 4 et l'horloge 6.

Le circuit de commande opératoire fournit les signaux EA1, EB1 et EC1 respectivement aux circuits 31 et 4. La commande opératoire donne le signal dit de départ S1 à SN après envoi de l'impulsion d'émission. On actionne ainsi un compteur de temps de retard 111. Le compteur 111 est chargé par son entrée EA1 à une valeur initiale qui est décomptée jusque la valeur zéro où il émet un signal de zéro. Ce signal, composé avec le signal C1 fournit par l'horloge 6 dans une porte ET, transmet le signal C1 d'horloge à un diviseur 121. Le diviseur 121 divise la fréquence du signal d'horloge C1 par un facteur Z. Le signal à fréquence divisée est transmis à un compteur de longueur de ligne 131 qui a été porté par la commande 2 par son entrée EB1 à une valeur fixe. Le compteur 131 permet selon la position d'un commutateur électronique la mise en cadence du signal de mesure X1 par l'intermédiaire de la logique 51 dans la ligne à retard 71. Ce commutateur électronique est ici constitué par une bascule 14 dans le circuit 4 de commande d'entrée-sortie. La bascule 14 engendre un signal B1 à BN transmis à la logique 51 d'entré-sortie. Le signal B1 est composé par l'intermédiaire de quatre portes ET au signal A1 fournit des bascules 15 et 16.

Après l'arrêt du compteur de longueur de ligne 131, le signal utile est complètement enregistré dans l'une des CCD 71A ou 71B de la ligne à retard 71. Et la cadence s'arrête. C'est seulement lorsque tous les compteurs de longueur de ligne 131 à 13N sont remis à zéro que l'on peut extraire l'information utile en appliquant le signal de libération F. Quand tous les compteurs de ligne 131 sont à zéro, un élément de commutation électronique, ici réalisé par une bascule 15, s'inverse et demeure en cet état tant qu'un compteur de sortie est en déroulement. Pendant ce temps, les CCD qui ont reçu auparavant le signal utile sont vidés en parallèle. Quand ils sont tous vides, la cadence s'arrête.

Pour gérer l'ensemble des canaux, il est possible avec le commutateur 14 et le commutateur 15 de libérer l'un ou l'autre des groupes de CCD A ou B, c'est-à-dire, libérer les CCD 71A à 7NA puis, de préparer l'autre groupe pour l'entrée d'un nouveau signal utile 71B à 7NB. On peut donc ainsi commencer déjà un nouveau cycle d'entrée dès que les compteurs sont remis à l'état initial. Le compteur de sortie 17 n'est remis à cet état qu'après achèvement de la sortie.

Un autre commutateur existant dans la commande d'entrée-sortie 4, par exemple une bascule 16, permet que l'impulsion soit assurée à la sortie. Il commute sur le front descendant du signal provenant de l'horloge pilote 9 de l'horloge 6. La fréquence de cette horloge a été divisée par le secteur Z/2 dans un diviseur 10. On est ainsi assuré que, quel que soit l'instant d'inversion du commutateur électronique 15, le cadencement du signal utile se fait sur un flan du signal d'horloge et non à l'instant du changement d'état du commutateur 15. L'emploi du signal de fréquence d'horloge divisé d'un facteur Z/2 donne l'assurance que l'imprécision du temps de retard sera seulement de l'ordre d'un cycle de la fréquence pilote 9.

L'emploi de deux CCD 71A et 71B est avantageux en ce que tout en sortant une précédente information utile, on peut simultanément mais après avoir retardé en conséquence le début de l'enregistrement faire entrer une information utile nouvelle. On y trouve encore aussi un avantage par rapport au cadencement connu à fréquence variable d'horloge ne comportant qu'un CCD par canal. En effet, la fréquence d'horloge pour la sortie de l'information utile précédente est la même que celle nécessaire pour l'entrée de l'information utile suivante. Une erreur de phase apparaîtrait lors de la sommation de l'information utile suivante.

D'autres éléments à transfert de charge peuvent être utilisés ainsi que les circuits à couplage de charge CCD. Des technologies spéciales permettent à l'heure actuelle d'enterrer dans l'élément à transfert de charge un canal par lequel le transfert des charges, à la commande, est facilité. Des éléments dits PCCD (en langue anglaise Peristaltic Charge Coupled Device) sont bien connus pour leur rapidité de fonctionnement (jusqu'à cent mégahertz). Ils trouvent application dans l'invention où ils permettent de réaliser des débits d'informations plus rapides.

## Revendications

1. Dispositif récepteur pour échographe comprenant une sonde ultrasonore comportant une pluralité d'éléments transducteurs (11-1N), un nombre correspondant d'unités d'éléments à transfert de charge (71-7N) interconnectés respectivement entre chaque transducteur et un moyen sommateur (8), lesdites unités étant destinées à emmagasiner des signaux d'écho délivrés selon une séquence de réception prédéterminée par les éléments transducteurs, et un circuit de contrôle (2, 4, 6, 31-3N, 51-5N) pilotant la mémorisation et la lecture des signaux d'écho emmagasinés et déterminant, pour chaque unité, un délai de déclenchement de la mémorisation, ledit circuit comportant une horloge (6), caractérisé en ce que pour chaque unité (71, 7N) le circuit de contrôle comporte un moyen retardateur (111, 11N), une porte (P2) et un compteur de longueur de ligne (131) déterminant la durée pendant laquelle les signaux d'écho sont admis à l'unité associée, chaque moyen retardateur (111, 11N) étant connecté entre la dite horloge et la porte (P2) associée dont la sortie est reliée à une entrée de commande de l'unité associée ainsi qu'au compteur de longueur de ligne (131) associé, le circuit de contrôle comportant en outre un circuit de commande opératoire (2) délivrant des codes numériques pour programmer à chaque séquence, d'une part, chacun desdits moyens retardateurs et, d'autre part, chacun desdits compteurs de longueur de ligne, chaque moyen retardateur et chaque compteur de longueur de ligne comportant à cet effet des entrées de programmation respectives, lesdits codes numériques appliqués à chaque séquence étant respectivement représentatifs de l'instant initial de chargement des éléments à transfert de charge de ladite unité associée et de la durée des signaux d'écho à emmagasiner dans lesdits éléments, et une porte de validation aux entrées de laquelle les sorties (D1, Dn) de tous les compteurs de longueur de ligne sont reliées, ainsi que des moyens de lecture simultanée (4, 51, 5N) des éléments à transfert de charge préalablement utilisés en chargement-écriture, lesdits moyens de lecture étant pilotés par la porte de validation.

2. Dispositif selon la revendication 1, caractérisé en ce que chaque unité d'éléments à transfert de charge comporte deux tels éléments en parallèle entre l'élément transducteur correspondant et ledit moyen sommateur (8) et en ce qu'il comporte des moyens (4, 51-5N) pour valider alternativement l'un des éléments à transfert de charge en chargement-écriture pour recevoir un signal utile généré par ledit élément transducteur et l'autre en déchargement-lecture pour appliquer un signal utile précédent audit moyen sommateur.

3. Dispositif selon la revendication 1, caractérisé en ce qu'il comporte pour chaque unité, un diviseur numérique (121) interconnecté entre ladite porte et ladite unité.

## Claims

1. Reception device for an echograph comprising an ultrasonic probe having a plurality of transducer elements (11-1N), a corresponding number of charge transfer device units (71-7N) interconnected respectively between each transducer and a summation means (8), said device units being intended to store the echo signals delivered in predetermined reception sequence by the transducer elements, and a control circuit (2, 4, 6, 31-3N, 51-5N) controlling the storing and the reading of the stored echo signals and determining for each unit a release delay of the storing, said circuit comprising a clock (6), characterized in that for each unit (71, 7N) the control circuit comprises delay means (111, 11N), a gate (P2) and a line length counter (131) determining the duration during which the echo signals are admitted to the associated unit, each delay means (111, 11N) being connected between said clock and the associated gate (P2) whose output is connected to a control input of the associated unit and also to the associated line length counter (131), the control circuit further comprising an operation control circuit (2) delivering digital codes for programming in each sequence on the one hand each of said delay means and on the other hand each of said line length counters, each delay means and each line length counter comprising for this purpose respective programming inputs, said digital codes applied in each sequence being respectively representative of the initial instant of charging of the charge transfer elements of said associated unit and of the duration of the echo signals to be stored in said elements and an enabling gate to the inputs of which the outputs (D1, Dn) of all the line length counters are connected, as well as means (4, 51, 5N) for simultaneous reading of the charge transfer elements previously used in charge write mode, said reading means being controlled by the enabling gate.

2. Device according to claim 1, characterized in that each charge transfer device unit comprises two such elements in parallel between the corresponding transducer element and said summation means (8) and in that it comprises means (4, 51-5N) for enabling alternatively one of the charge transfer elements in charge-write mode for receiving a useful signal generated by said transducer element and the other in discharge-read mode for applying a preceding useful signal to said summation means.

3. Device according to claim 1, characterized in that it includes for each unit a digital divider (121) interconnected between said gate and said unit.

**Patentansprüche**

1. Empfangsvorrichtung für einen Echograph, mit einer Ultraschallsonde, welche mehrere Wandlerelemente (11-1N), eine entsprechende Anzahl von Einheiten von Ladungsüberführungselementen (71-7N), die jeweils zwischen einen Wandler und eine Summiereinrichtung (8) geschaltet sind, wobei die genannten Einheiten dazu bestimmt sind, die Echosignale zu speichern, welche in einer vorbestimmten Empfangsfolge von den Wandlerelementen abgegeben werden, und eine Steuerschaltung (2, 4, 6, 31-3N, 51-5N) enthält, welche die Speicherung und das Auslesen der gespeicherten Echosignale steuert und für jede Einheit eine Auslöseverzögerung der Einspeicherung bestimmt, wobei diese Schaltung eine Zeitbasis (6) umfaßt, dadurch gekennzeichnet, daß für jede Einheit (71, 7N) die Steuerschaltung eine Verzögerungseinrichtung (111, 11N), eine Torschaltung (P2) und einen Leitungslängenzähler (131) umfaßt, welcher die Dauer bestimmt, während welcher die Echosignale Zugang zu der zugeordneten Einheit haben, wobei jede Verzögerungseinrichtung (111, 11N) zwischen die genannte Zeitbasis und die zugeordnete Torschaltung (P2) geschaltet ist, deren Ausgang mit einem Steuereingang der zugeordneten Einheit sowie mit dem zugeordneten Leitungslängenzähler (131) verbunden ist, wobei die Steuerschaltung ferner eine Operations-Steuerschaltung (2) umfaßt, welche digitale Codes ausgibt, um in jeder Folge einerseits jede der Verzögerungseinrichtungen und andererseits jeden der Leitungslängenzähler zu programmieren, wobei jede Verzögerungseinrichtung und jeder Leitungslängenzähler zu diesem Zweck entsprechende Programmiereingänge aufweist, wobei ferner die digitalen, in jeder Folge angewendeten Codes jeweils den Anfangszeitpunkt des Ladens der Ladungsüberführungselemente der zugeordneten Einheit und die Dauer der in die genannten Elemente einzuspeichernden Echosignale repräsentieren, und eine Freigabeschaltung, an deren Eingänge die Ausgänge (D1, Dn) aller Leitungslängenzähler angeschlossen sind, sowie Mittel zum gleichzeitigen Auslesen (4, 51, 5N) der Ladungsüberführungselemente umfaßt, welche zuvor im Lade-Schreib-Betrieb verwendet wurden, wobei die genannten Lesemittel durch die Freigabeschaltung gesteuert werden.

2. Vorrichtung nach Anspruch 1, dadurch gekennzeichnet, daß jede Einheit von Ladungsüberführungselementen zwei derartige Elemente in Parallelschaltung zwischen dem entsprechenden Wandlerelement und der genannten Summiereinrichtung (8) umfaßt und daß sie Mittel (4, 51-5N) umfaßt, um abwechselnd eines der Ladungsüberführungselemente für den Lade-Schreib-Betrieb zum Empfang eines durch das genannte Wandlerelement erzeugten Nutzsignals und das andere zum Entlade-Lese-Betrieb freizugeben, um ein vorausgehendes Nutzsignal an die genannte Summiereinrichtung anzulegen.

3. Vorrichtung nach Anspruch 1, dadurch gekennzeichnet, daß sie für jede Einheit einen digitalen Teiler (121) umfaßt, welcher zwischen die genannte Torschaltung und die genannte Einheit eingefügt ist.

# FIG_1

LOGIQUE D'ENTREE-SORTIE

FIG_2

0 054 045